# EUROPÄISCHE PATENTANMELDUNG

(11) **EP 1 394 548 A2**
(43) Veröffentlichungstag der Anmeldung: **03.03.2004**
(21) Anmeldenummer: 03014303.6
(22) Anmeldetag: 09.02.1996
(51) Int. Cl.: G01N 33/576, G01N 33/68, C07K 14/18

(54) **Methode zur serologischen Typisierung mittels typspezifischer Antigene**

(30) Priorität: 09.02.1995 DE 19504302
(62) Teilanmeldung aus: 96101925.4
(71) Anmelder: Roche Diagnostics GmbH, 68305 Mannheim (DE)
(72) Erfinder: Seidel, Christoph, Dr., 82362 Weilheim (DE); Wienhues-Thelen, Ursula-Henrike, Dr., 82152 Krailling (DE); Schmitt, Urban, 82386 Oberhausen (DE)
(74) Vertreter: Weiss, Wolfgang, Dipl.-Chem. Dr.

(57) **Zusammenfassung**

Die Erfindung betrifft ein Verfahren zur Typisierung von Antikörpern in einer Probeflüssigkeit mittels typspezifischer Antigene, insbesondere ein Verfahren zur Typisierung von Antikörpern gegen Hepatitis C-Virus und hierfür geeignete Peptidantigene.

## Beschreibung

Die Erfindung betrifft ein Verfahren zur Typisierung von Antikörpern in einer Probeflüssigkeit mittels typspezifischer Antigene, insbesondere ein Verfahren zur Typisierung von Antikörpern gegen Hepatitis C-Virus und hierfür geeignete Peptidantigene.

Die als Non-A-non-B-Hepatitis bezeichnete Krankheit wird in vielen Fällen durch das Hepatitis C-Virus (HCV) hervorgerufen. Bei HCV handelt es sich um ein einzelsträngiges verkapseltes RNA-Virus, dessen Genom aus etwa 9000 bis 10000 Basen besteht. Von diesem Genom werden Strukturproteine (Kern- und Hüllproteine) sowie Nicht-Strukturproteine codiert.

Bei HCV handelt es sich um einen Virus von großer klinischer Bedeutung, da er mit chronischen Infektionen und spät auftretenden Erkrankungen von Infizierten, wie etwa kryptische Zirrhose und primäres Leberkarzinom in Korrelation steht.

Die Übertragung von HCV kann durch Blutkontakt erfolgen. Untersuchungen zum Auftreten von Antikörpern lassen eine leichte Übertragbarkeit vermuten.

EP-A-0 318 216 offenbart eine partielle Nukleotidsequenz eines HCV. EP-A-O 450 931 offenbart die vollständige Nukleotid- und Aminosäuresequenz eines HCV. Verfahren zum diagnostischen Nachweis einer HCV-Infektion durch Bestimmung viraler Antikörper in Körperflüssigkeiten unter Verwendung von viralen Proteinen oder Peptiden als Antigene sind bekannt (vgl. z.b. Mori et al., Jpn. J. Cancer Res. 83 (1992), 264-268; WO92/11370 und DE-A-44 28 705.4).

Bei einer Infektion mit HCV besteht das Problem, daß unterschiedliche Virenstämme existieren, die eine erhebliche Variabilität in ihrem Genom und entsprechend in den von diesem Genom codierten Polypeptiden aufweisen (vgl. z.B. McOmish et al., Bioforum 16 (1993), 414-420).

Aufgrund der Variabilität von HCV besteht einerseits die Schwierigkeit, eine Infektion überhaupt zu diagnostizieren und andererseits den für die Infektion verantwortlichen Virenstamm richtig zu typisieren. Eine derartige Typisierung ist wichtig, da sich verschiedene Virenstämme in ihrer Virulenz und in ihrem Ansprechen auf eine Therapie, z.B. mit Interferon, deutlich unterscheiden.

Eine Möglichkeit zur Typisierung von Virenstämmen besteht in einer Bestimmung des Genotyps durch Amplifizierung des viralen Genoms mittels PCR und anschließender Sequenzbestimmung (z.B. Bukh et al., Proc. Natl. Acad. Sci. USA 90 (1993), 8234-8238). Ein Nachteil dieser Genotypbestimmung besteht jedoch darin, daß die Schritte der Amplifizierung und Sequenzbestimmung nur aufwendig und unter Verwendung komplizierter Apparaturen in speziell dafür ausgerüsteten Laboratorien durchführbar sind. Dies gilt umso mehr, da bei einer HCV-Infektion die Menge an amplifizierbarem viralem genetischem Material oft nur extrem gering ist.

Eine weitere Möglichkeit zur Typbestimmung ist die Serotypisierung, d.h. die Bestimmung des Virustyps über die immunologische Spezifität der im Organismus gegen den Virus erzeugten Antikörper. Simmonds et al. (J. Clin. Microbiol. 31 (1993), 1493-1503) beschreiben die Verwendung von typspezifischen Peptidantigenen zur serologischen Differenzierung von Infektionen mit den HCV-Typen 1, 2 und 3. Die Typisierung erfolgte mittels eines indirekten ELISA unter Verwendung von Peptidantigenen der Aminosäurebereiche 1691 - 1708 und 1710 - 1728 aus der NS4-Region von HCV. Hierzu wurden typspezifische Peptidantigene jeweils gesondert nach Typ in einzelnen Löchern einer Mikrotiterplatte immobilisiert und mit jeweils gesonderten Aliquots einer Plasmaprobe von HCV-infizierten Blutspendern in Kontakt gebracht. Die Typisierung erfolgte entsprechend der Reaktivität der Serumprobe mit den einzelnen Peptidantigenen. Dieses Verfahren ist jedoch relativ ungenau und erlaubt überdies keine Bestimmung einzelner viraler Subtypen, d.h. einzelner Virenstämme, die sich in ihrer Immunogenität nur in geringem Umfang unterscheiden.

Das der vorliegenden Erfindung zugrundeliegende Problem bestand somit darin, ein neues Verfahren zur Typisierung von Antikörpern bereitzustellen, welches einerseits routinemäßig und ohne großen apparativen Aufwand durchführbar ist und das andererseits eine ausreichend genaue Klassifizierung zwischen einzelnen Antikörpertypen ermöglicht. Ein weiteres, der vorliegenden Erfindung zugrundeliegendes Problem war, Regionen aus dem Genom von HCV zu identifizieren, die gleichzeitig eine hohe Immunogenität und Variabilität besitzen, so daß sie sich zur Typisierung von HCV-Infektionen eignen.

Dieses Problem wird gelöst durch ein fraktioniertes Immunsorptionsverfahren, bei dem ein erstes Aliquot einer Probeflüssigkeit, welche die zu typisierenden Antikörper enthält, nacheinander mit einer Reihe typspezifischer Antigene bzw. Antigengemische in Kontakt gebracht wird und gegebenenfalls ein zweites Aliquot oder weitere Aliquots der Probeflüssigkeit ebenfalls - aber in jeweils anderer Reihenfolge - mit verschiedenen typspezifischen Antigenen bzw. Antigengemischen in Kontakt gebracht werden. Weiterhin werden neue immunogen wirksame Peptidsequenzen aus dem HCV-Genom bereitgestellt, die eine bessere Typisierung als die aus dem Stand der Technik bekannten Sequenzen ermöglichen.

Ein erster Aspekt der vorliegenden Erfindung ist ein Verfahren zur Typisierung von Antikörpern in einer Probeflüssigkeit, welches dadurch gekennzeichnet ist, daß man
(a) ein erstes Aliquot der Probeflüssigkeit mit einem ersten immobilisierten Antigen, das spezifisch für einen ersten Typ der zu untersuchenden Antikörper ist, oder einem ersten Gemisch von immobilisierten Antigenen, die jeweils spezifisch für einen ersten Typ der zu untersuchenden Antikörper sind, unter Bedingungen in Kontakt bringt, wobei eine Reaktion des Antigens oder Antigengemisches mit den Antikörpern stattfinden kann und wobei die Antikörpermenge in der Probeflüssigkeit die Kapazität des immobilisierten Antigens oder Antigengemisches nicht überschreitet,
(b) die Probeflüssigkeit aus Schritt (a) mit einem zweiten immobilisierten Antigen, das spezifisch für einen zweiten Typ der zu untersuchenden Antikörper ist, oder einem Gemisch von immobilisierten Antigenen, die jeweils spezifisch für einen zweiten Typ der zu untersuchenden Antikörper sind, unter Bedingungen wie in Schritt (a) in Kontakt bringt, wobei das zweite Antigen oder Antigengemisch räumlich getrennt von dem in Schritt (a) verwendeten ersten Antigen oder Antigengemisch ist,
(c) die Maßnahmen gemäß Schritt (b) gegebenenfalls mit einem oder mehreren weiteren Antigenen oder Antigengemischen wiederholt, die spezifisch für einen oder mehrere weitere Typen der zu untersuchenden Antikörper sind, wobei die weiteren Antigene oder Antigengemische jeweils räumlich getrennt von den in vorherigen Schritten verwendeten Antigenen oder Antigengemischen sind,
(d) ein zweites Aliquot der Probeflüssigkeit gegebenenfalls mit mehreren immobilisierten Antigenen oder Antigengemischen entsprechend den Schritten (a) bis (c) in Kontakt bringt, wobei aber die Reihenfolge der Antigene oder Antigengemische eine andere ist,
(e) die jeweilige immunologische Reaktivität der immobilisierten Antigene oder Antigengemische mit der Probeflüssigkeit qualitativ oder/und quantitativ bestimmt und
(f) auf Basis der Reaktivitätsbestimmung eine Typisierung der in der Probeflüssigkeit vorhandenen Antikörper vornimmt.

Als Typisierungsobjekte können beliebige Antikörper untersucht werden, z.B. Antikörper, die gegen Pathogene oder Autoimmunantigene gerichtet sind. Die Typisierung der Antikörper ermöglicht wiederum eine Typisierung der Antigene, denen der Organismus ausgesetzt war und die die Bildung der Antikörper hervorgerufen haben. Vorzugsweise werden Antikörper typisiert, die gegen ein oder mehrere Pathogene gerichtet sind, insbesondere Antikörper, die gegen virale Antigene gerichtet sind. Beispiele für Viren, von denen die viralen Antigene stammen, sind HCV, Human-Papillomavirus (HPV), Hepatitis B-Virus (HBV) und HIV. Das Verfahren kann auch für den parallelen Nachweis mehrerer nebeneinander vorliegender viraler Infektionen, z.B. HCV und HIV, verwendet werden.

Besonders bedeutsam ist der Einsatz des erfindungsgemäßen Verfahrens für die Typsisierung einer HCV-Infektion, da sich die einzelnen Virustypen und Subtypen bezüglich ihrer Virulenz und dem Ansprechen gegenüber einer Interferontherapie unterscheiden. Doch auch bei anderen Viren, wie etwa HIV, kann das Verfahren vorteilhaft dazu eingesetzt werden, die Herkunft einzelner viraler Isolate zu bestimmen bzw. Subtypen (z.B. Subtyp O bei HIV) zu identifizieren.

Beim erfindungsgemäßen Verfahren wird ein Aliquot der Probeflüssigkeit, z.B. einer gegebenenfalls verdünnten Körperflüssigkeit wie etwa Blut, Plasma, Serum oder Urin, nacheinander mit mehreren immobilisierten typspezifischen Antigenen oder Antigengemischen in Kontakt gebracht, um eine schrittweise Sorption der mit den jeweiligen Antigenen oder Antigengemischen reaktionsfähigen Antikörper und damit eine schrittweise Entfernung aus der Probeflüssigkeit zu ermöglichen. Vorzugsweise werden parallel dazu ein oder mehrere weitere Aliquots der Probeflüssigkeit mit den immobilisierten Antigenen oder Antigengemischen in einer anderen Reihenfolge als das erste Aliquot in Kontakt gebracht. Bei Verwendung von zwei Aliquots der Probeflüssigkeit wird das zweite Aliquot mit den immobilisierten Antigenen bzw. Antigengemischen vorzugsweise in umgekehrter Reihenfolge als das erste Aliquot in Kontakt gebracht.

Um eine möglichst quantitative Sorption der Antikörper eines Typs an das jeweilige Antigen zu erreichen, soll die Antikörpermenge in der Probeflüssigkeit die Kapazität der immobilisierten Antigene nicht überschreiten. Dies kann auf einfache Weise durch entsprechende Verdünnungen der Probeflüssigkeit erreicht werden. Aufgrund der aufeinanderfolgenden Sorptionsschritte mit mehreren verschiedenen typspezifischen immobilisierten Antigenen oder Antigengemischen ist das erfindungsgemäße Verfahren eine fraktionierte Immunsorption.

Für die einzelnen Sorptionsschritte des erfindungsgemäßen Verfahrens werden vorzugsweise typspezifische Antigengemische eingesetzt. Typspezifische Antigengemische sind Gemische von Antigenen, die aus den gleichen Bereichen einzelner Varianten innerhalb eines zu klassifizierenden Antigentyps stammen und untereinander nur geringe Unterschiede verglichen mit anderen zu klassifizierenden Antikörpertypen aufweisen, oder/und Gemische von Antigenen, die aus unterschiedlichen Bereichen des gleichen Antigentyps stammen.

Die immobilisierten typspezifischen Antigene, mit denen die Probeflüssigkeit in Kontakt gebracht wird, können beliebige Antigene sein, sofern sie eine Typisierung der damit reaktionsfähigen Antikörper erlauben. Vorzugsweise sind die Antigene Peptidsequenzen, die einen immunologisch aktiven Bereich von mindestens 6 Aminosäuren enthalten. Vorzugsweise besitzt ein immunologisch aktiver Bereich eine Länge von maximal 30 Aminosäuren und besonders bevorzugt eine Länge von 9 bis 20 Aminosäuren.

Neben einem immunologisch aktiven Bereich kann das Peptid vorzugsweise noch einen Spacerbereich enthalten, der beispielsweise zur Kopplung mit anderen immunologisch aktiven Bereichen oder einem Träger oder/und zur Kopplung von Markierungs- bzw Festphasenbindungsgruppen dienen kann.

Der Spacerbereich ist vorzugsweise eine immunologisch inaktive Peptidsequenz mit einer Länge von 1 bis 10 Aminosäuren. Die Aminosäuren des Spacerbereichs werden vorzugsweise ausgewählt aus natürlichen oder artifiziellen Aminosäuren, insbesondere aus Aminosäuren der Gruppe, bestehend aus Glycin, ß-Alanin, γ-Aminobuttersäure, ∈-Aminocapronsäure und Lysin. Der Spacerbereich ist vorzugsweise eine kontinuierliche Abfolge von Aminosäuren am Amino- oder/und Carboxyterminus des immunologisch aktiven Epitopbereichs.

Die immobilisierten Antigene können an beliebige Festphasen gebunden sein, z.B. an die Wand oder/und den Boden eines Reaktionsgefäßes, an Säulen oder auch an partikuläre Festphasen. Besonders bevorzugt werden auf Mikrotiterplatten immobilisierte Antigene eingesetzt.

Die Immobilisierung der Antigene an die Festphase kann auf an sich beliebige Weise erfolgen. In einer bevorzugten Ausführungsform des erfindungsgemäßen Verfahrens tragen die Antigene eine Festphasenbindungsgruppe, über die sie mittels einer Affinitätswechselwirkung an eine reaktive Festphase gekoppelt sind. Vorzugsweise wird die Festphasenbindungsgruppe aus Biotin oder Biotinderivaten, wie etwa Iminobiotin oder Desthiobiotin, ausgewählt, die an eine mit Streptavidin oder Avidin beschichtete Festphase binden können. Andere Beispiele für geeignete Festphasenbindungsgruppen sind Haptene, wie etwa Dinitrophenol, Digoxin, Digoxigenin etc., die an einer mit einem Hapten-spezifischen Antikörper bedeckten Festphase binden können.

Andererseits können die Antigene auch kovalent mit der Festphase verknüpft sein, z.B. über einen bifunktionellen Spacer. Schließlich können die Antigene auch an einen Träger konjugiert vorliegen, der adsorptiv an der Festphase gekoppelt ist. Beispiele für geeignete Träger sind Proteinmoleküle, wie etwa Rinderserumalbumin. Weitere Verfahren zur Immobilisierung von Antigenen und insbesondere von Peptidantigenen auf einer Festphase sind dem Fachmann bekannt und brauchen daher nicht näher erläutert zu werden.

Die qualitative oder/und quantitative Bestimmung der Reaktivität der in der Probe vorhandenen Antikörper mit den immobilisierten Antigenen kann auf jede bekannte Weise erfolgen, z.B. durch Inkubation mit einem markierten Zweitantikörper, der Spezies-spezifisch einen Antikörper aus der Probeflüssigkeit erkennen kann (z.B. ein Ziege-Anti-human-Antikörper). Die Typisierung der Antikörper erfolgt auf Basis der Reaktivitätsbestimmungen. Wenn eine Typisierung von Antikörpern duchgeführt werden soll, die gegen sehr ähnliche Antigene gerichtet sind (z.B. virale Subtypen), ist es bevorzugt, wenn ein weiteres Aliquot der Probeflüssigkeit mit den zu immobilisierten Antigenen oder Antigengemischen in anderer Reihenfolge als das erste Aliquot in Kontakt gebracht wird (Schritt (d) des erfindungsgemäßen Verfahrens). Ergibt sich dann bei beiden Testrichtungen der gleiche Subtyp, kann eine eindeutige Unterscheidung getroffen werden.

Die erfindungsgemäße Methode der fraktionierten Immunsorption erlaubt eine erhebliche Zeitersparnis, Einsparung von Probenmaterial, Reaktionsgefäßen, Antigenen und Inkubationspuffer im Vergleich zur Methode des Standes der Technik, bei der eine Vorinkubation verschiedener Seren mit typheterologen Peptiden in jeweils gesonderten, nicht mit Streptavidin beschichteten ELISA-Platten erfolgt. Weiterhin vereinigt die neue Methode den Vorteil einer Doppelbestimmung mit dem einer Vorinkubation des Probenmaterials, ohne hierbei zusätzliches Probenmaterial zu verbrauchen.

Das erfindungsgemäße Verfahren eignet sich insbesondere zur Typisierung von Antikörpern gegen Hepatitis C-Virus (HCV). Für diese Typisierung werden Antigene benötigt, die gleichzeitig zwei Voraussetzungen erfüllen, nämlich eine immunogene Wirkung, d.h. die Fähigkeit zur Bildung von Antikörpern hervorzurufen, die gegen diese Sequenzen gerichtet sind und weiterhin eine Variabilität bei einzelnen Virustypen bzw. -subtypen, durch die eine Unterscheidung der einzelnen Virenisolate überhaupt erst möglich wird.

Überraschenderweise wurden Peptidsequenzen aus dem Genom von HCV identifiziert, die diese beiden Voraussetzungen hervorragend erfüllen. Diese Peptidsequenzen eignen sich zur Herstellung von Peptidantigenen für ein Verfahren zur Bestimmung von Antikörpern gegen Hepatitis C-Virus.

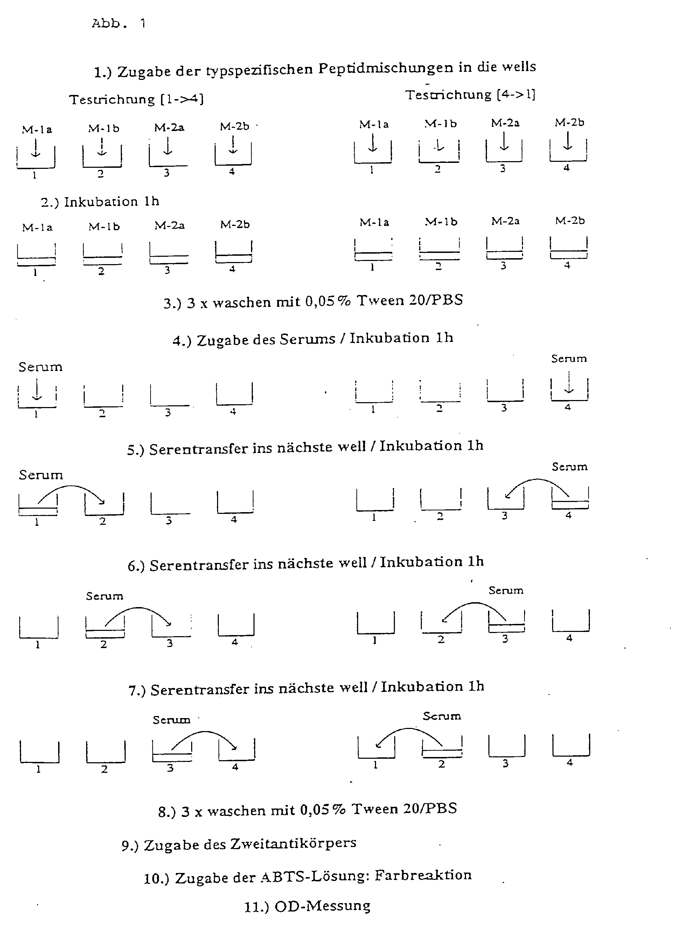

Ein weiterer Gegenstand der vorliegenden Erfindung ist somit ein Peptid, umfassend mindestens einen immunologisch aktiven Bereich aus dem Hepatitis C-Virus, der ausgewählt ist aus
(a) den Aminosäuren 384 - 414,
(b) den Aminosäuren 1738 - 1759,
(c) den Aminosäuren 2217 - 2236,
(d) den Aminosäuren 2402 - 2419,
(e) den Aminosäuren 2345 - 2357
und Teilsequenzen davon, die eine Länge von mindestens 6 Aminosäuren aufweisen, wobei sich die Numerierung der Aminosäurereste auf Fig. 1 von EP-A-0 450 931 bezieht.

Das erfindungsgemäße Peptid kann aus einem beliebigen HCV-lsolat stammen, beispielsweise aus einem HCV-lsolat mit der in EP-A-0 450 931 beschriebenen Nukleotidsequenz.

Wenn das Peptid aus dem Bereich der Aminosäuren 384 - 414 stammt, der in der hypervariablen Region lokalisiert ist, wird der immunologisch aktive Bereich vorzugsweise aus (a) den in SEQ ID NO. 1 bis 10 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren ausgewählt.

Unter dem Begriff "Homologie" wird im Sinne der vorliegenden Anmeldung ein prozentualer Wert verstanden, der sich ergibt, wenn man die Zahl identischer Aminosäuren zweier zu vergleichender Aminosäuresequenzen durch die Anzahl aller Aminosäuren einer von beiden Sequenzen dividiert.

Die Sequenzprotokolle SEQ ID NO. 1 bis 3 zeigen HCV-Sequenzen aus der hypervariablen Region von Virusisolaten des Typs 1a. SEQ ID NO. 4 bis 6 zeigen Sequenzen von Virusisolaten des Typs 1 b. SEQ ID NO. 7 zeigt eine Sequenz eines Virusisolats des Typs 2a. SEQ ID NO. 8 und 9 zeigen Sequenzen von Virusisolaten des Typs 2b. SEQ ID NO. 1 bis 10 zeigen die Sequenz eines aus Taiwan stammenden Virusisolats.

Weiterhin kann der immunologisch aktive Bereich des Peptids aus den Aminosäuren 1738 - 1759 der NS4-Region und insbesondere aus den in SEQ ID NO. 11 bis 16 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Amionsäuren ausgewählt werden. SEQ ID NO. 11 und 12 zeigen Sequenzen von Virusisolaten des Typs 1a. SEQ ID NO. 13 zeigt eine Sequenz eines Virusisolats des Typs 1 b. SEQ ID NO. 14 zeigt eine Sequenz eines Virusisolats des Typs 2a. SEQ ID NO. 15 zeigt eine Sequenz eines Virusisolats des Typs 2b. SEQ ID NO. 16 zeigt eine Sequenz eines aus Taiwan stammenden Virusisolats.

Wenn das Peptid aus dem Bereich der Aminosäuren 2217 - 2236 der NS5-Region stammt, wird sein immunologisch aktiver Bereich vorzugsweise aus (a) den in SEQ ID NO. 17 bis 22 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren ausgewählt. SEQ ID NO. 17 zeigt die Sequenz eines Virusisolats des Typs 1a. SEQ ID NO. 18 und 19 zeigen Sequenzen von Virusisolaten des Typs 1b. SEQ ID NO. 20 zeigt die Sequenz eines Virusisolats des Typs 2a. SEQ ID NO. 21 zeigt die Sequenz eines Virusisolats des Typs 2b. SEQ ID NO. 22 zeigt die Sequenz eines aus Taiwan stammenden Virusisolats.

Wenn das Peptid aus den Aminosäuren 2402 - 2419 der NS5-Region stammt, wird sein immunologisch aktiver Bereich vorzugsweise aus (a) den in SEQ ID NO. 23 bis 24 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren ausgewählt. SEQ ID NO. 23 bis 24 zeigen Sequenzen von Virusisolaten der Typen 2a bzw. 2b.

Wenn das Peptid aus dem Bereich der Aminosäuren 2345 - 2357 der NS5-Region stammt, wird der immunologisch aktive Bereich aus (a) den in SEQ ID NO. 25 bis 30 dargestellten Aminosäuresequenzen, (b) Aminosäuresequenzen, die eine Homologie von mindestens 90 % zu einer der Sequenzen aus (a) besitzen oder (c) Teilsequenzen der Sequenzen aus (a) oder (b) mit einer Länge von mindestens 6 Aminosäuren ausgewählt. SEQ ID NO. 25 und 26 zeigen Sequenzen von Virusisolaten des Typs 1a.

SEQ ID NO. 27 zeigt eine Sequenz eines Virusisolats des Typs 1b. SEQ ID NO. 28 und 29 zeigen Sequenzen von Virusisolaten der Typen 2a bzw. 2b. SEQ ID NO. 30 zeigt die Sequenz eines aus Taiwan stammenden Virusisolats.

Vorzugsweise weist der immunologisch aktive Bereich der Peptide eine Länge von maximal 30 Aminosäuren, besonders bevorzugt eine Länge von 9 bis 20 Aminosäuren auf. Neben dem immunologisch aktiven HCV-Peptidbereich kann das Peptid weiterhin einen immunologisch inaktiven Spacerbereich wie oben definiert umfassen.

Weiterhin kann das erfindungsgemäße Peptid mindestens eine Festphasenbindungsgruppe tragen, die vorzugsweise aus Biotin und Biotinderivaten ausgewählt ist. Andererseits kann das Peptid jedoch auch eine Markierungsgruppe tragen. Die Markierungsgruppe kann eine beliebige radioaktive oder nicht-radioaktive Markierungsgruppe sein. Die bevorzugten nicht-radioaktiven Markierungsgruppen können direkt oder/und indirekt nachweisbar sein. Bei einer direkt nachweisbaren Markierung ist die ein nachweisbares Meßsignal erzeugende Gruppe direkt auf dem Peptidantigen lokalisiert. Beispiele für solche direkt signalerzeugenden Gruppen sind Chromogene (fluoreszierende oder lumineszierende Gruppen, Farbstoffe), Enzyme, NMR-aktive Gruppen oder Metallpartikel, die auf bekannte Weise an ein Peptidantigen gekoppelt sind. Vorzugsweise ist die direkt nachweisbare Markierungsgruppe ein durch Elektrochemilumineszenz nachweisbarer Metallkomplex, besonders bevorzugt ein Rutheniumkomplex. Geeignete Metallkomplexe sind beispielsweise in EP-A-0 580 979, WO90/05301, WO90/11511 und WO92/14138 beschrieben. Auf diese Dokumente wird hiermit Bezug genommen.

Eine andere Art der Markierung ist die indirekt nachweisbare Markierung. Bei dieser Art der Markierung ist das Peptidantigen mit einer indirekt nachweisbaren Gruppe gekoppelt, z.B. einer Haptengruppe, die wiederum durch Reaktion mit einem geeigneten Bindepartner (z.B. Anti-Hapten-Antikörper), der wiederum eine signalerzeugende Gruppe trägt, nachweisbar ist.

Die oben beschriebenen neuen Peptide können in einem Verfahren zur Bestimmung von Antikörpern gegen Hepatitis C-Virus verwendet werden. Einerseits können sie als Antigene in einem diagnostischen Verfahren zum Nachweis einer HCV-Infektion eingesetzt werden, z.B. in einem Doppelantigenbrückentest, bei dem eine Probeflüssigkeit mit mindestens zwei Peptiden P1 und P2 inkubiert wird, wobei das Peptid P1 (a) an eine Festphase gebunden ist oder (b) in einer an eine Festphase bindefähigen Form vorliegt und das Peptid P2 eine Markierungsgruppe trägt. Der Antikörper in der Probeflüssigkeit wird durch Bestimmung der Markierung in der Festphase oder/und in der flüssigen Phase, vorzugsweise in der Festphase, über einen immobilisierten Immunkomplex nachgewiesen. Bei einem derartigen Verfahren zum Nachweis einer HCV-Infektion werden als Antigene vorzugsweise Peptidgemische verschiedener Typen bzw. Subtypen von HCV eingesetzt, so daß eine HCV-Infektion unabhängig von ihrem Typ bzw. Subtyp eindeutig nachgewiesen werden kann.

Andererseits können die erfindungsgemäßen Peptide auch in einem Verfahren zur Typisierung von Antikörpern gegen HCV eingesetzt werden, wobei das Typisierungsverfahren vorzugsweise nach der Methode der fraktionierten Immunsorption durchgeführt wird.

In diesem Zusammenhang ist anzumerken, daß die Typisierung von HCV-Antikörpern nach der Methode der fraktionierten Immunsorption nicht nur unter Verwendung der obigen Peptide durchgeführt werden kann, sondern daß auch Peptide aus anderen Sequenzbereichen des HCV-Genoms geeignet sind, z.B. die in Beispiel 2 angegebenen Peptide oder Teilsequenzen davon mit einer Länge von mindestens 6 Aminosäuren.

Das erfindungsgemäße Verfahren soll durch die folgenden Beispiele, Abbildungen und Sequenzprotokolle näher erläutert werden.

Es zeigen:
Abb. 1 ein Schema für die Durchführung einer Typisierung nach der Methode der fraktionierten Immunsorption,
SEQ ID NO. 1 bis 10 Aminosäuresequenzen verschiedener Hepatitis C-Virusisolate im Bereich der Aminosäuren 384 - 414,
SEQ ID NO. 11 bis 16 Aminosäuresequenzen verschiedener Hepatitis C-Virusisolate im Bereich der Aminosäuren 1738 - 1759,
SEQ ID NO. 17 bis 22 Aminosäuresequenzen verschiedener Hepatitis C-Virusisolate im Bereich der Aminosäuren 2217 - 2236,
SEQ ID NO. 23 bis 24 Aminosäuresequenzen verschiedener Hepatitis C-Virusisolate aus den Aminosäuresequenzen 2402 - 2419 und
SEQ ID NO. 25 bis 30 Aminosäuresequenzen verschiedener Hepatitis C-Virusisolate aus dem Bereich der Aminosäuren 2345 -2357.

### Beispiel 1

Synthese von Biotin-Peptidamiden aus fünf unterschiedlichen Bereichen des HCV-Polyproteins und deren Verwendung zur Typisierung von HCV-Seren

Die synthetisierten Teilbereiche wurden so ausgewählt, daß sie sich durch eine geringe Sequenhomologie zwischen den Isolaten der Typen 1a, 1b, 2a, 2b und Taiwan (gehört zu Typ 1b) der entsprechenden Region auszeichnen, wodurch die hergestellten Peptide entsprechend der verschiednene HCV-Typen für die serologische Typisierung verwendet werden können. Zudem können durch die typübergreifende Synthese der entsprechenden Regionen eventuelle Erkennungslücken in der HCV-Diagnostik durch die Testung von Peptiden, die nicht dem bisher stets als Grundlage dienenden HCV-1-lsolat gemäß EP-A-0 450 931 zuzordnen sind, schließen.

Als besondere Regionen wurden die hypervariable Region mit zum Teil sehr starkem Aminosäureaustausch sowie Regionen, bei denen bestimmte HCV-Isolate eine Insertion bzw. Deletion in ihrer Sequenz gegenüber den anderen Isolaten erfahren, ausgewählt.

### Testvorschrift

Getestet wurden 24 Seren, wobei der ELISA-Test wie folgt ablief:
1.) Beschichtung einer Streptavidin-ELISA-Platte mit 100 µl Peptidlösung (Konz. 50ng/100 µl), 1 h
2.) 3 x Waschen mit 0,05 % Tween 20/PBS
3.) Inkubation mit 100 µl Serum (1:100 verdünnt), 1 h
4.) 3 x Waschen mit 0,05 % Tween 20/PBS
5.) Inkubation mit 150 µl eines markierten Zweitantikörpers (Ziege-Anti-Human-Antikörper-Peroxidase-Konjugat, 1:10000 verdünnt), 1 h
6.) ABTS®-Farbreaktion, 1 h
7.) OD-Messung (positive HCV-Detektion ab 200 mOD)

Es wurden folgende Biotin-Peptidamide aus unterschiedlichen Regionen des HCV-Polyproteins gegen 24 Seren getestet.
1. E2/NS1 -Region (hypervariable Region) [AS 384-414]: Peptide gemäß SEQ ID NO. 1 - 10
2. NS4-Region [AS 1738 - 1759]: Peptide gemäß SEQ ID NO. 11-16
3. NS5-Region [AS 2217 - 2236, Deletion]: Peptide gemäß SEQ ID NO. 17 - 22
4. NS5-Region [AS 2402 - 2419, Insertion]: Peptide gemäß SEQ ID NO. 23 - 24
5. NS5-Region [AS 2345 - 2357, Deletion]: Peptide gemäß SEQ ID NO. 25 - 30

### Ergebnis:

Die getesteten Peptide (SEQ ID NO. 1 - 30) erwiesen sich zur Typisierung von HCV-positiven Seren geeignet. Insbesondere die Peptide aus der hypervariablen Region waren den aus dem Stand der Technik (Simmonds et al., J. Clin. Microbiol. 31 (1993), 1493-1503) bekannten Peptiden überlegen.

### Beispiel 2

Serologische Typisierung von HCV-Seren mit der Methode der fraktionierten Immunsorption

Für die Typisierung von HCV-Seren wurde eine neue Methode entwickelt, die auf einer fraktionierten Immunsorption beruht.

Die Typisierung wurde unter Verwendung von Peptidmischungen durchgeführt, die nur subtypspezifische Biotin-Peptidamide enthalten:
M-1a: enthält nur Peptide des Typs 1a
M-1b: enthält nur Peptide des Typs 1b
M-2a: enthält nur Peptide des Typs 2a
M-2b: enthält nur Peptide des Typs 2b.

Die Testdurchführung ist schematisch in Abb. 1 dargestellt.

### Test in Richtung [1 -> 4]:

1.) Beschichtung von 4 wells eines Streptavidin-beschichteten Mikrotiterplatte mit je 100 µl der subtypspezifischen Peptidmischungen M-1a (well 1), M-1b (well 2), M-2a (well 3) und M-2b (well 4), 1 h.
2.) 3 x Waschen mit 0,05% Tween 20/PBS
3.) Zugabe von 100 µl des 1:100 verdünnten Serums in well 1, Inkubation 1 h
4.) Quantitative Überführung der 100/µl Serum von well 1 nach well 2, Inkubation 1 h
5.) Quantitative Überführung der 100 µl Serum von well 2 nach well 3, Inkubation 1 h
6.) Quantitative Überführung der 100 µl Serum von well 3 nach well 4, Inkubation 1 h
7.) 3 x Waschen sämtlicher 4 wells mit 0,05 % Tween 20/PBS
8.) Zugabe von 150 µl Zweitantikörper (Ziege-anti-human-POD-Konjugat, Verdünnung 1:10000) in alle 4 wells, Inkubation 1 h
9) 3 x Waschen mit 0,05 % Tween 20/PBS
10) Farbreaktion: Zugabe von 150 µl ABTS®-Lösung, Inkubation 1 h
11) OD-Messung

Bezüglich der Vorinkubation des Serums läßt sich folgendes feststellen: Kommt das Serum in das well 4 (Peptidmischung M-2b), d.h. Test des Serums auf Typ 2b, dann hat es die maximale Vorinkubation durch die Peptide der Typen 1 a (well 1), 1 b (well 2) und 2a (well 3) erfahren. Entsprechend geringer ist die Vorinkubation des Serums, wenn es auf Typ 2a (Vorinkubation durch Peptide der Typen 1a und 1b) und auf Typ 1b (Vorinkubation durch Peptide des Typs 1a) getestet wird. Bei der OD-Messung des wells 1 (Test des Serums auf Typ 1a) wird das Serum somit keiner typheterologen Vorinkubation unterzogen.

Um nun zu vermeiden, daß die Reihenfolge des "Serentransfers" (well 1 -> 2 -> 3 -> 4), d.h. der Grad der Vorinkubation, einen Einfluß auf die Testergebnisse hat, wird das Prinzip des "Serentransfers" zusätzlich in umgekehrter Reihenfolge vorgenommen (well 4 -> 3 -> 2 -> 1). Gekoppelt an den obigen Test [1->4] wird parallel der Test [4->1] durchgeführt:
Durchführung wie in Richtung [1->4], nur daß die Serumprobe zuerst in well 4, dann in well 3, dann in well 2 und schließlich in well 1 gegeben wurde.

### Bezüglich der Vorinkubation des Serums läßt sich analog folgendes feststellen:

Kommt das Serum in das well 1 (Peptidmischung M-1a), d.h. Test des Serums auf Typ 1a, dann hat es die maximale Vorinkubation durch die Peptide der Typen 2b (well 4), 2a (well 3) und 1b (well 2) erfahren. Entsprechend geringer ist die Vorinkubation des Serums, wenn es auf Typ 1 b (Vorinkubation durch Peptide der Typen 2b und 2a) und auf Typ 2a (Vorinkubation durch Peptide des Typs 2b) getestet wird. Bei der OD-Messung des wells 4 (Test des Serums auf Typ 2b) wird das Serum keiner typheterologen Vorinkubation unterzogen.

### Verwendete Biotin-Peptidamide:

| Core 4-Region: | |
|---|---|
| A (HCV-1): Biotin - PIPKA RRPEG RTWAQ PGY-NH₂ MW: 2689,19 g/Mol; | Typ 1 (a+b) |
| B (HCV-J6): Biotin - PIPKD RRSTG KSWGK PGY-NH₂ MW: 2639,13 g/Mol; | Typ 2 (a+b) |

| E1-Region: | |
|---|---|
| C (HCV-1): Biotin - ATRDGKLPATQLRRHIDLLKG-NH₂ MW: 2968,57 g/Mol; | Typ 1a |
| D (HCV-J): Biotin - AARNSSIPTTTIRRHVDLLVG-NH2 MW: 2886,41 g/Mol; | Typ 1b |
| E (HCV-J6): Biotin - AVQQPGALTQGLRTHIDMVVM-NH₂ MW: 2874,49 g/Mol; | Typ 2a |
| F (HCV-J7/J8): Biotin - AVKHRGALTRSLRTHVDMIVM-NH₂ | Typ 2b |
| | MW: 3001,71 g/Mol |

| NS 4/1-Region: | |
|---|---|
| G (HCV-1): Biotin - S Q H L P Y l E Q - NH₂ MW: 1723,02 g/Mol; | Typ 1 a |
| H (HCV-J): Biotin - A S H L P Y l E Q - NH₂ MW: 1664,86 g/Mol; | Typ 1 b |
| l (HCV-J6): Biotin - A S R A A L I E E - NH₂ MW: | Typ 2a |
| J (HCV-J8): Biotin - A S K A A L I E E - NH₂ MW: 1538,84 g/Mol; | Typ 2b |

| NS 4/2-Region: | |
|---|---|
| K (HCV-I): Biotin - QKALGLLQT-NH₂ MW: 1578,92 g/Mol; | Typ 1a |
| L (HCV-J): Biotin - SKIQGLLQQ-NH₂ MW: 1621,93 g/Mol; | Typ 1b |

| NS 5/1-Region: | |
|---|---|
| M (HCV-1): Biotin - SRRFAQALPVWARPD-NH₂ MW: 2378,83 g/Mol; | Typ 1a |
| N (HCV-J): Biotin - PRKFPPALPIWARPD-NH₂ MW: 2369,90 g/Mol; | Typ 1b |
| O (HCV-J6): Biotin - KKRFPPALPAWARPD-NH₂ MW: 2358,89 g/Mol; | Typ 2a |
| P (HCV-J8): Biotin - RRKFPPALPPWARPD-NH₂ MW: 2412,94 g/Mol; | Typ 2b |

### Peptidmischungen:

- M-1 a:: 100 µl M-1a bestehen aus je 20 µl der Peptide A, C, G, K und M (Konz.: 2.5 µg/ml, d.h. 50 ng/20µl)
[4 ml der Mischung M-1a bestehen aus je 400 µl Peptidlösung obiger 5 Peptide (Konz.: 5 µg/ml) + 2 ml Puffer]
- M-1b:: 100 µl M-1b bestehen aus je 20 µl der Peptide A, D, H, L und N (Konz.: 2,5 µg/ml, d.h. 50 ng/20 µl)
[4 ml der Mischung M-2b bestehen aus je 400//I Peptidlösung obiger 5 Peptide (Konz.: 5 µg/ml) + 2 ml Puffer]
- M-2a:: 100 µl M-2a bestehen aus je 25 µl der Peptide B, E, l und O (Konz.: 2 µg/ml, d.h. 50 ng/25 µl)
[4 ml der Mischung M-2b bestehen aus je 400 µl Peptidlösung obiger 4 Peptide (Konz.: 5 µg/ml) + 2,4 ml Puffer]
- M-2b:: bestehen aus je 25 µl der Peptide B, F, J und P (Konz.: 2 µg/ml, d.h. 50 ng/25 µl)
[4 ml der Mischung M-2b bestehen aus je 400 µl Peptidlösung obiger 4 Peptide (Konz.: 5 µg/ml) + 2,4 ml Puffer]

### Ergebnis:

12 HCV-positive Seren wurden mit Hilfe der Methode der fraktionierten Immunsorption typisiert.

Von den 12 Seren konnten 11 mit Hilfe der verwendeten Peptidpanele typisiert werden, während bei einem Serum keine Reaktivität (d.h. Extinktion < 200 mOD bei allen Typen) feststellbar war, wodurch eine Typisierung auch tendenziell nicht möglich war.

**Tab 2:**

| Typisierung von 11 HCV-Seren mit Peptidmischungen jeweils in beiden Testrichtungen | | | |
|---|---|---|---|
| Seren | Typisierung | | Ergebnis der Typisierung |
| | Test [1 - > 4] | Test [4- > 1 ] | |
| 1 | Typ 1a | Typ 1a | Typ 1a |
| 2 | Tendenz 1a | Tendenz 1b | Tendenz 1 |
| 3 | Typ 1a | Typ 1b | Typ 1 |
| 4 | Typ 1a | Tendenz 1b | Typ 1 |
| 5 | Typ 1a | negativ | Typ 1 |
| 6 | Typ 1a | Typ 1b | Typ 1 |
| 7 | Typ 1a | Tendenz 2 | Tendenz Typ 1 |
| 8 | Typ 1a | Typ 1b | Typ 1 |
| 9 | Typ 1a | Typ 1a | Typ 1a |
| 10 | Typ 1a | Typ 1b | Typ 1 |
| 11 1 | Typ 1a | Tendenz 1b | Typ 1 |

Bei einer Reaktivität < 200 mOD wurde entweder bei Dominanz eines Typs die Bewertung "Tendenz ..." vorgenommen oder bei unklarem Ergebnis "negativ" bewertet.

Neben der Aussage über die Typzugehörigkeit (d.h. Typ 1, 2 oder 3) des Serums durch die obig durchgeführte Typisierungsmethode, zeigte das Ergebnis, daß bei einigen Seren sogar eine Typisierung im Hinblick auf den Subtyp (1a, 1b, 2a, 2b) möglich ist (Seren 1 und 9). Dieser Fall liegt dann vor, wenn beide Testrichtungen, d.h. Test [1->4] und Test [4->1], den gleichen Subtyp ergeben.

## Patentansprüche

1. Peptid, umfassend mindestens einen immunologisch aktiven Bereich aus dem Hepatitis C-Virus, der ausgewählt ist aus
(a) den Aminosäuren 384 - 414,
(b) den Aminosäuren 1738 - 1759,
(c) den Aminosäuren 2217 - 2236,
(d) den Aminosäuren 2402 - 2419,
(e) den Aminosäuren 2345 - 2357
und Teilsequenzen davon, die eine Länge von mindestens 6 Aminosäuren aufweisen.

2. Peptid nach Anspruch 1,
**dadurch gekennzeichnet,**
**daß** der immunologisch aktive Bereich ausgewählt ist aus (a) den in SEQ ID NO. 1 - 10 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren.

3. Peptid nach Anspruch 1,
**dadurch gekennzeichnet,**
**daß** der immunologisch aktive Bereich ausgewählt ist aus (a) den in SEQ ID NO. 11 - 16 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren.

4. Peptid nach Anspruch 1,
**dadurch gekennzeichnet,**
**daß** der immunologisch aktive Bereich ausgewählt ist aus (a) den in SEQ ID NO. 17 - 22 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren.

5. Peptid nach Anspruch 1,
**dadurch gekennzeichnet,**
**daß** der immunologisch aktive Bereich ausgewählt ist aus (a) den in SEQ ID NO. 23 - 24 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren.

6. Peptid nach Anspruch 1,
**dadurch gekennzeichnet,**
**daß** der immunologisch aktive Bereich ausgewählt ist aus (a) den in SEQ ID NO. 25 - 30 dargestellten Aminosäuresequenzen, (b) Aminosäuresequenzen, die eine Homologie von mindestens 90 % zu einer der Sequenzen aus (a) besitzen, oder (c) Teilsequenzen der Sequenzen aus (a) oder (b) mit einer Länge von mindestens 6 Aminosäuren.

7. Verwendung von Peptiden nach einem der Ansprüche 1 bis 6 in einem Verfahren zur Bestimmung von Antikörpern gegen Hepatitis C-Virus.


(1) ALGEMEINE INFORMATION:

(i) ANMELDER:
(A) NAME: Boehringer Mannheim GmbH
(B) STRASSE: Sandhofer Strasse 112 - 132
(C) ORT: Mannheim
(E) LAND: DE
(F) POSTLEITZAHL: 68305

(ii) ANMELDETITEL: Methode zur serologischen Typisierung mittels
typspezifischer Antigene

(iii) ANZAHL DER SEQUENZEN: 30

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 1:

Glu Thr His Val Thr Gly Gly Ser Ala Gly His Thr Val Ser Gly Phe
1               5                   10                  15

Val Ser Leu Leu Ala Pro Gly Ala Lys Gln Asn Val Gln Leu Ile
            20                  25                  30

(2) INFORMATION ZU SEQ ID NO: 2:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 2:

Glu Thr His Val Thr Gly Gly Asn Ala Gly Arg Thr Thr Ala Gly Leu
1               5                   10                  15

Val Gly Leu Leu Thr Pro Gly Ala Lys Gln Asn Ile Gln Leu Ile

            20                  25                  30

(2) INFORMATION ZU SEQ ID NO: 3:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 3:

His Thr Arg Val Thr Gly Gly Val Gln Gly His Val Thr Ser Thr Leu
1               5                   10                  15

Thr Ser Leu Phe Arg Pro Gly Ala Ser Gln Lys Ile Gln Leu Val
            20                  25                  30

(2) INFORMATION ZU SEQ ID NO: 4:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 4:

His Thr His Val Thr Gly Gly Arg Val Ala Ser Ser Thr Gln Ser Leu
1               5                   10                  15

Val Ser Trp Leu Ser Gln Gly Pro Ser Gln Lys Ile Gln Leu Val
            20                  25                  30

(2) INFORMATION ZU SEQ ID NO: 5:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 5:

Asp Thr His Val Thr Gly Gly Ala Gln Ala Lys Thr Thr Asn Arg Leu
1               5                   10                  15

Val Ser Met Phe Ala Ser Gly Pro Ser Gln Lys Ile Gln Leu Ile
            20                  25                  30

(2) INFORMATION ZU SEQ ID NO: 6:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 6:

Glu Thr His Val Thr Gly Gly Ser Ala Gly His Thr Val Ser Gly Phe
1               5                   10                  15

Val Ser Leu Leu Ala Pro Gly Ala Lys Gln Asn Val Gln Leu Ile
            20                  25                  30

(2) INFORMATION ZU SEQ ID NO: 7:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 7:

Gln Thr His Thr Val Gly Gly Ser Thr Ala His Asn Ala Arg Thr Leu
1               5                   10                  15

Thr Gly Met Phe Ser Leu Gly Ala Arg Gln Lys Ile Gln Leu Ile
            20                  25                  30

(2) INFORMATION ZU SEQ ID NO: 8:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 8:

Thr Thr Tyr Ser Ser Gly Gln Glu Ala Gly Arg Thr Val Ala Gly Phe
1               5                   10                  15

Ala Gly Leu Phe Thr Thr Gly Ala Lys Gln Asn Leu Tyr Leu Ile
            20                  25                  30

(2) INFORMATION ZU SEQ ID NO: 9:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 9:

Ser Thr Gln Val Thr Gly Gly Gln Ala Ala His Thr Val Arg Gly Val
1               5                   10                  15

Ala Ser Ile Phe Ser Pro Gly Ser Arg Gln Asp Ile Ser Leu Ile
            20                  25                  30

(2) INFORMATION ZU SEQ ID NO: 10:

(i) SEQUENZ CHARAKTERISTIKA:
(A) LÄNGE: 31 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 10:

Ser Thr Ile Val Ser Gly Gly Thr Val Ala Arg Thr Thr His Ser Leu
1               5                   10                  15

Ala Ser Leu Phe Thr Gln Gly Ala Ser Gln Lys Ile Gln Leu Ile
            20                  25                  30

(2) INFORMATION ZU SEQ ID NO: 11:

(i) SEQUENZ CHARAKTERISTIKA:
(A) LÄNGE: 22 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 11:

Thr Ala Ser Arg Gln Ala Glu Val Ile Ala Pro Ala Val Gln Thr Asn
1               5                   10                  15

Trp Gln Lys Leu Glu Thr
            20

(2) INFORMATION ZU SEQ ID NO: 12:

(i) SEQUENZ CHARAKTERISTIKA:

(A) LÄNGE: 22 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 12:

Thr Ala Ser Arg His Ala Glu Val Ile Thr Pro Ala Val Gln Thr Asn
1               5                   10                  15

Trp Gln Lys Leu Glu Val
            20

(2) INFORMATION ZU SEQ ID NO: 13:

(i) SEQUENZ CHARAKTERISTIKA:
(A) LÄNGE: 22 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 13:

Thr Ala Thr Lys Gln Ala Glu Ala Ala Ala Pro Val Val Glu Ser Lys
1               5                   10                  15

Trp Arg Ala Leu Glu Val
            20

(2) INFORMATION ZU SEQ ID NO: 14:

(i) SEQUENZ CHARAKTERISTIKA:
(A) LÄNGE: 22 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 14:

Gln Ala Ser Lys Gln Ala Gln Asp Ile Gln Pro Ala Val Gln Ala Ser
1               5                   10                  15

Trp Pro Lys Val Glu Gln
            20

(2) INFORMATION ZU SEQ ID NO: 15:

(i) SEQUENZ CHARAKTERISTIKA:
(A) LÄNGE: 22 Aminosäuren

(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 15:

Gln Ala Thr Arg Gln Ala Gln Asp Ile Gln Pro Ala Ile Gln Ser Ser
1               5                   10                  15

Trp Pro Lys Leu Glu Gln
            20

(2) INFORMATION ZU SEQ ID NO: 16:

(i) SEQUENZ CHARAKTERISTIKA:
(A) LÄNGE: 22 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 16:

Thr Ala Thr Lys Gln Ala Glu Ala Ala Ala Pro Val Val Glu Ser Lys
1               5                   10                  15

Trp Arg Thr Leu Glu Ala
            20

(2) INFORMATION ZU SEQ ID NO: 17:

(i) SEQUENZ CHARAKTERISTIKA:
(A) LÄNGE: 20 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 17:

Ala Asn His Asp Ser Pro Asp Ala Glu Leu Ile Glu Ala Asn Leu Leu
1               5                   10                  15

Trp Arg Gln Glu
            20

(2) INFORMATION ZU SEQ ID NO: 18:

(i) SEQUENZ CHARAKTERISTIKA:
(A) LÄNGE: 20 Aminosäuren
(B) ART: Aminosäure

(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 18:

Thr His His Asp Ser Pro Asp Ala Asp Leu Ile Glu Ala Asn Leu Leu
1               5                   10                  15

Trp Arg Gln Glu
            20

(2) INFORMATION ZU SEQ ID NO: 19:

(i) SEQUENZ CHARAKTERISTIKA:
(A) LÄNGE: 20 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 19:

Thr His His Val Ser Pro Asp Ala Asp Leu Ile Glu Ala Asn Leu Leu
1               5                   10                  15

Trp Arg Gln Glu
            20

(2) INFORMATION ZU SEQ ID NO: 20:

(i) SEQUENZ CHARAKTERISTIKA:
(A) LÄNGE: 16 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 20:

Thr His Gly Lys Ala Tyr Asp Val Asp Met Val Asp Ala Asn Leu Phe
1               5                   10                  15

(2) INFORMATION ZU SEQ ID NO: 21:

(i) SEQUENZ CHARAKTERISTIKA:
(A) LÄNGE: 16 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 21:

Thr His Lys Thr Ala Tyr Asp Cys Asp Met Val Asp Ala Asn Leu Phe
1               5                   10                  15

(2) INFORMATION ZU SEQ ID NO: 22:

(i) SEQUENZ CHARAKTERISTIKA:
(A) LÄNGE: 20 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 22:

Thr Arg His Thr Pro Pro Asp Ala Asp Leu Ile Glu Ala Asn Leu Leu
1               5                   10                  15

Trp Arg Gln Glu
            20

(2) INFORMATION ZU SEQ ID NO: 23:

(i) SEQUENZ CHARAKTERISTIKA:
(A) LÄNGE: 18 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 23:

Pro Glu Gln Val Glu Leu Gln Pro Pro Pro Gln Gly Gly Val Val Thr
1               5                   10                  15

Pro Gly

(2) INFORMATION ZU SEQ ID NO: 24:

(i) SEQUENZ CHARAKTERISTIKA:
(A) LÄNGE: 18 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 24:

Phe Glu Pro Val Gly Ser Ala Pro Pro Ser Glu Gly Glu Cys Glu Val
1               5                   10                  15

Ile Asp

(2) INFORMATION ZU SEQ ID NO: 25:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 25:

Ala Glu Leu Ala Thr Arg Ser Phe Gly
1               5

(2) INFORMATION ZU SEQ ID NO: 26:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 26:

Ala Glu Leu Ala Thr Lys Ser Phe Gly
1               5

(2) INFORMATION ZU SEQ ID NO: 27:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 27:

Ala Glu Leu Ala Thr Lys Thr Phe Gly
1               5

(2) INFORMATION ZU SEQ ID NO: 28:

(i) SEQUENZ CHARAKTERISTIKA:
(A) LÄNGE: 13 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 28:

Gln Gln Leu Ala Ile Lys Ser Phe Gly Gln Pro Pro Pro
1               5                   10

(2) INFORMATION ZU SEQ ID NO: 29:

(i) SEQUENZ CHARAKTERISTIKA:
(A) LÄNGE: 13 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 29:

Arg Glu Met Ala Asp Lys Val Leu Ser Pro Leu Gln Asp
1               5                   10

(2) INFORMATION ZU SEQ ID NO: 30:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(vi) URSPRÜNLICHE HERKUNFT:
(A) ORGANISMUS: Hepatitis C Virus

(xi) SEQUENZBESCHREIBUNG: SEQ ID NO: 30:

Ala Glu Leu Ala Thr Lys Thr Phe Gly
1               5